# EUROPÄISCHE PATENTSCHRIFT

(11) **EP 2 243 023 B1**
(45) Veröffentlichungstag und Bekanntmachung des Hinweises auf die Patenterteilung: **17.05.2017**
(21) Anmeldenummer: 09709583.0
(22) Anmeldetag: 09.02.2009
(51) Int. Cl.: G01N 27/28, G01N 27/27, B01L 3/00, G01N 33/543

(54) **VORRICHTUNG UND VERFAHREN MIT EINEM SENSOR-ARRAY UND MIT EINEM PORÖSEN STEMPEL SOWIE DEREN VERWENDUNG**
APPARATUS AND METHOD COMPRISING A SENSOR ARRAY AND A POROUS PLUNGER, AND USE THEREOF
DISPOSITIF ET PROCÉDÉ COMPRENANT UN RÉSEAU DE CAPTEURS ET UN PISTON POREUX ET UTILISATION DE CEUX-CI

(30) Priorität: 15.02.2008 DE 102008009184
(43) Veröffentlichungstag der Anmeldung: 27.10.2010
(73) Patentinhaber: Boehringer Ingelheim Vetmedica GmbH, 55216 Ingelheim am Rhein (DE)
(72) Erfinder: GUMBRECHT, Walter, 91074 Herzogenaurach (DE); PAULICKA, Peter, 91056 Erlangen (DE); STANZEL, Manfred, 91056 Erlangen (DE)
(74) Vertreter: Von Rohr Patentanwälte Partnerschaft mbB
(86) Internationale Anmeldenummer: PCT/EP2009/051428
(87) Internationale Veröffentlichungsnummer: WO 2009/101041

(56) Entgegenhaltungen:
- WO-A-02/43937
- WO-A-2006/097751
- DE-C1- 10 058 394
- US-A1- 2002 164 820
- US-B1- 6 921 514

## Beschreibung

Die vorliegende Erfindung bezieht sich auf eine Vorrichtung mit einem Sensor-Array und mit einem Stempel, und auf ein Verfahren unter Verwendung der Vorrichtung zum Nachweis von Flüssigkeiten oder Substanzen aus Flüssigkeiten in räumlich verschiedenen Reaktionsbereichen. Eine entsprechende Vorrichtung sowie ein Verfahren zu deren Betrieb gehen aus der DE 100 58 394 C1 hervor. Die Erfindung betrifft ferner deren Verwendung in einer Chipkarte.

In der Sensorik werden vermehrt Sensor-Arrays eingesetzt, um mehrere chemische Verbindungen gleichzeitig detektieren zu können. Speziell in der Bioanalytik werden diese Sensor-Arrays verwendet, z.B. zur DNA-Analyse, zum Nachweis von Krankheitserregern oder für die Untersuchung von Stoffwechselstörungen. Dabei werden Körperflüssigkeiten wie z.B. Blut oder Urin durch chemische Reaktionen aufgearbeitet. Danach werden diese über die Sensor-Arrays geleitet und durch optische, Frequenz- oder elektrochemische Messungen werden Wechselwirkungen zwischen den Sensoren der Sensor-Arrays und den nachzuweisenden Substanzen aus den Körperflüssigkeiten untersucht. Die Sensoren der Sensor-Arrays sind dabei häufig mit Molekülen beschichtet, welche spezifisch mit den nachzuweisenden Substanzen aus den Körperflüssigkeiten reagieren. Durch Waschen mit Flüssigkeiten können ungebundene Substanzen von den Oberflächen der Sensoren entfernt werden. Die nachzuweisenden, spezifisch gebundenen Substanzen können dann direkt oder indirekt, z.B. über Marker, nachgewiesen werden.

Typische Nachweismethoden umfassen optische Messungen, Frequenz-Messungen oder elektrochemische Messungen. So können z.B. über Ellipsometrie die Änderungen der Brechungseigenschaften der Oberfläche nach Anbindung der nachzuweisenden Substanzen untersucht werden. Eine Alternative ist das Anbinden von spezifischen, optisch aktiven Markern an die nachzuweisenden Substanzen. Diese können z.B. über ihre Fluoreszenz oder sichtbare Farbe bei Bestrahlung mit bestimmten Wellenlängen nachgewiesen werden. Frequenzmessungen mit einer Quarz-Mikrowaage können Änderungen der Masse von an der Oberfläche gebundenen Molekülen nachweisen. Strom-SpannungsMessungen mit Hilfe von Elektroden in einer Flüssigkeit können elektrochemisch das Anbinden der nachzuweisenden Substanzen, oder deren angebundenen Zustand direkt oder über Reaktionsprodukte nachweisen.

Die beschriebenen Methoden haben gemeinsam, dass unbestimmte Strömungsverhältnisse über den Sensoren zu Messfehlern führen können. Abhängig von den Strömungen bilden sich an den Grenzschichten zwischen den Sensor-Oberflächen und der Flüssigkeit unterschiedliche Doppelschichten aus oder binden unspezifisch Moleküle an die Oberflächen, wodurch sich z.B. die Reaktionsfähigkeit, die Bindungs-Affinität, die optischen, elektrischen und Reibungs-Eigenschaften der Sensor-Oberflächen ändern.

Das Erzeugen von konstanten Strömungen über den Sensoren der Sensor-Arrays ist aufwendig und erfordert einen hohen Apparativen Aufwand. Deshalb wird bevorzugt bei stationären Flüssigkeiten gemessen, d.h. ohne störende Strömungen über den Sensoren.

Im Stand der Technik, vergleiche z.B. die Druckschrift DE 100 58 394 C1, ist eine Anordnung und ein Verfahren beschrieben, um stationäre Flüssigkeiten über Sensoren von Sensor-Arrays zu erzeugen. Die Sensoren eines Sensor-Arrays sind auf einer planaren Oberfläche angeordnet mit Wänden zwischen den einzelnen Sensoren, wobei die Wände aus der Oberfläche herausragen. Parallel zur planaren Oberfläche des Sensor-Arrays ist beabstandet zu den Wänden ein Gehäuseoberteil angeordnet, welches genügend Abstand zu der Oberfläche und den Wänden aufweist, um Flüssigkeit zwischen der Oberfläche und dem Gehäuseoberteil strömen zu lassen. Vor der Messung wird mit Hilfe eines Stempels das Gehäuseoberteil in Richtung Oberfläche mit Wänden gedrückt, so dass die Wände und das Gehäuseoberteil in engem Kontakt stehen.

Das Strömen der Flüssigkeit ist unterbunden, und über den Sensoren sind abgeschlossene Reaktionsräume, begrenzt durch die planare Oberfläche, die Wände und das Gehäuseoberteil, entstanden. Die Flüssigkeit, welche in den Reaktionsräumen eingeschlossen ist, kann nun ohne Flüssigkeitsaustausch zwischen verschiedenen Reaktionsräumen untersucht werden. Die Messung der Sensoren findet mit Hilfe der abgeschlossenen Reaktionsräume unabhängig voneinander statt.

Das im Stand der Technik gemäß der DE 100 58 394 C1 beschriebene Verfahren führt zu einer Anordnung mit Gehäuseoberteil und Stempel, bei der die Wände zwischen den Sensoren genauen Abmessungen entsprechen müssen. Diese genauen Abmessungen sind in der Praxis schwer zu realisieren:
Ragen die Wände zu wenig aus der planaren Oberfläche heraus, in welcher die Sensoren angeordnet sind, so entstehen zu kleine Reaktionsräume mit zu wenig Flüssigkeitsmenge, um eine aussagekräftige Messung durchzuführen. Das Gehäuseoberteil kann unter Umständen, bei hohem Stempeldruck auf den Sensoren aufsitzen, womit die Messung verfälscht wird.

Sind die Wände zu groß dimensioniert, so müssen die Sensoren in einem größeren Abstand voneinander angeordnet werden, und die nötige Flüssigkeitsmenge zum Befüllen der Anordnung steigt. Damit fällt die Empfindlichkeit der Messung geringer aus und die Zahl der zur Messung zur Verfügung stehenden Sensoren sinkt bei gleicher Fläche, da diese größer dimensioniert werden müssen um eine ausreichende Messgenauigkeit zu erreichen.

Aufgabe der Erfindung ist es, eine im Vergleich zum Stand der Technik einfachere, weniger fehleranfälligere und kostengünstigere Vorrichtung sowie Verfahren und deren Anwendung zum Nachweis von Flüssigkeiten oder Substanzen in Flüssigkeiten anzugeben. Insbesondere ist es Aufgabe der Erfindung, die Abhängigkeit der Messgenauigkeit und der Zuverlässigkeit der Vorrichtung von den Fehlertoleranzen der Fertigung der Wände zwischen den Sensoren zu reduzieren. Ein einfacherer Aufbau mit weniger Fehlermöglichkeiten soll Kosten bei der Herstellung der Vorrichtung sparen und die Messgenauigkeit erhöhen. Die Möglichkeiten der Beschädigung der Vorrichtung im Gebrauch sollen reduziert werden.

Die angegebene Aufgabe wird bezüglich der Vorrichtung mit den Merkmalen des Anspruchs 1, bezüglich des Verfahrens mit den Merkmalen des Anspruchs 13, bezüglich der Verwendung der Vorrichtung und des Verfahrens mit den Merkmalen des Anspruchs 21 gelöst.

Vorteilhafte Ausgestaltungen der erfindungsgemäßen Vorrichtung, des Verfahrens und deren Verwendung gehen aus den jeweils zugeordneten abhängigen Unteransprüchen hervor. Dabei können die Merkmale der nebengeordneten Ansprüche mit Merkmalen eines jeweils zugeordneten Unteranspruchs oder vorzugsweise auch mit Merkmalen mehrerer zugeordneter Unteransprüche kombiniert werden.

Die erfindungsgemäße Vorrichtung für den Nachweis von Flüssigkeiten oder Substanzen aus Flüssigkeiten in räumlich voneinander getrennten Reaktionsräumen umfasst ein Sensor-Array und einen Stempel. Das Sensor-Array besteht aus wenigstens zwei Sensoren. Der Stempel weist eine Stempelschicht an seiner dem Sensor-Array gegenüberliegenden Oberfläche auf und ist beweglich gegenüber dem Sensor-Array angeordnet. Die Stempelschicht ist mit dem Sensor-Array mechanisch kontaktierbar. Sie weist zumindest teilweise oder vollständig poröse Eigenschaften auf und ist für Flüssigkeiten oder Substanzen aus Flüssigkeiten impermeabel bezogen auf die Gesamtschichtdicke der Stempelschicht.

In einer bevorzugten Ausführungsform der erfindungsgemäßen Vorrichtung sind die wenigstens zwei Sensoren auf oder in oder unter einer ersten Oberfläche angeordnet, insbesondere einer ersten planaren Oberfläche, und eine zweite Oberfläche, insbesondere eine zweite planare Oberfläche, ist von der Oberfläche der Stempelschicht gebildet, welche dem Sensor-Array gegenüber liegt.

In einer weiteren bevorzugten Ausführungsform ist der Stempel in einer ersten Position so anordenbar, dass die zweite Oberfläche einen Abstand zur ersten Oberfläche aufweist, insbesondere einen Abstand im Bereich von Mikrometern oder Millimetern, und in einer zweiten Position ist der Stempel so anordenbar, dass die zweite Oberfläche und die erste Oberfläche in direktem Kontakt stehen.

Bevorzugt weisen die Poren der porösen Stempelschicht einen Durchmesser im Bereich von 0,05 bis 100 Mikrometern auf, besonders bevorzugt im Bereich von 1 bis 10 Mikrometern.

Weiterhin bevorzugt ragen zwischen den mindestens zwei Sensoren Erhöhungen aus der ersten Oberfläche heraus, welche insbesondere in der Ebene der ersten Oberfläche die Sensoren jeweils vollständig umgeben oder umschließen, und deren Höhe, mit welcher die Erhöhungen aus der ersten Oberfläche herausragen, größer ist als jegliche Höhe von Strukturen, welche innerhalb von Teilen der ersten Oberfläche angeordnet sind, die von den Erhöhungen umgeben oder umschlossen sind.

Die Höhe und/oder Breite der Erhöhungen zwischen den mindestens zwei Sensoren ist bevorzugt in der Größenordnung der Durchmesser der Poren der Stempelschicht, insbesondere im Bereich von 0,05 bis 100 Mikrometern, besonders bevorzugt im Bereich von 1 bis 10 Mikrometern. Besonders bevorzugt sind in der zweiten Position des Stempels die aus der ersten Oberfläche herausragenden Erhöhungen teilweise oder vollständig in den Poren der Stempelschicht anordenbar.

In einer bevorzugten Ausführungsform der Vorrichtung besteht durch einen direkten Kontakt zwischen den Erhöhungen und der Stempelschicht eine für Gase oder Flüssigkeiten dichte Verbindung und/oder es ist nur ein Gas- oder Flüssigkeitsaustausch über jene Poren möglich, welche direkt über den Sensoren angeordneten sind.

In einer weiteren bevorzugten Ausführungsform der Vorrichtung ist der Raum zwischen dem Sensor-Array und der Stempelschicht teilweise oder vollständig mit Flüssigkeit füllbar oder gefüllt und/oder es sind nur Poren an der Oberfläche der porösen Stempelschicht mit Flüssigkeit füllbar oder gefüllt, wobei Poren im inneren der Stempelschicht keine Flüssigkeit enthalten und/oder nicht mit Flüssigkeit füllbar sind.

In einer besonders bevorzugten Ausführungsform der erfindungsgemäßen Vorrichtung sind die wenigstens zwei Sensoren elektrochemische Sensoren, insbesondere fingerförmig auf der ersten Oberfläche angeordnete Metallelektroden. Sie können aus Gold, Silber und/oder Silberchlorid, Platin, Palladium und/oder Kupfer aufgebaut sein, oder Verbindungen oder Legierungen dieser Metalle enthalten.

In einer weiteren bevorzugten Ausführungsform der Vorrichtung ist die poröse Stempelschicht aus einem porösen Material gebildet, welches porösen Kunststoff, porösen Gummi oder porösen Kautschuk enthält oder daraus gebildet ist. Insbesondere ist die poröse Stempelschicht aus einem Material, welches elastisch verformbar ist.

Die Schichtdicke der Stempelschicht ist bevorzugt im Bereich von Mikrometern, oder im Bereich von Millimetern, oder im Bereich von Zentimetern, und/oder der Stempel besteht vollständig aus der Stempelschicht.

Das erfindungsgemäße Verfahren zum Nachweis von Flüssigkeiten oder Substanzen aus Flüssigkeiten in räumlich voneinander getrennten Reaktionsräumen weist die zeitlich aufeinander folgenden Schritte auf,
- Befüllen und/oder Durchströmen eines Raumes mit einer Flüssigkeit, welcher gebildet wird vom Zwischenraum zwischen einer Oberfläche eines Sensor-Arrays, mit wenigstens zwei Sensoren, und einer Oberfläche einer Stempelschicht, welche zumindest teilweise oder vollständig poröse Eigenschaften aufweist und für Flüssigkeiten oder Substanzen aus Flüssigkeiten impermeabel ist bezogen auf die Gesamtschichtdicke der Stempelschicht,
- Bewegen oder Drücken der Stempelschicht in Richtung des Sensor-Arrays mit Hilfe eines mit der Stempelschicht in Verbindung stehenden Stempels, bis die Oberfläche des Sensor-Arrays mit der Oberfläche der Stempelschicht in direktem mechanischen Kontakt steht, und
- Messen mit den Sensoren des Sensor-Arrays.

Bevorzugt wird das Verfahren in der Form durchgeführt, dass während des direkten Kontaktes zwischen der Oberfläche des Sensor-Arrays und der Oberfläche der Stempelschicht kein Flüssigkeitsaustausch stattfindet, insbesondere zwischen jedem mit Flüssigkeit gefüllten Bereich direkt über einem ersten Sensor der wenigstens zwei Sensoren des Sensor-Arrays und jedem mit Flüssigkeit gefüllten Bereich direkt über dem wenigstens einem zweiten Sensor.

Besonders bevorzugt werden die mit Flüssigkeit befüllten Bereiche über den Sensoren ausschließlich durch Bereiche gebildet, welche aus mit Flüssigkeit gefüllten Poren an der Oberfläche der porösen Stempelschicht bestehen. Die Poren, welche nicht mit der Oberfläche der Stempelschicht in Verbindung stehen und im inneren der porösen Stempelschicht angeordnet sind, werden nicht mit Flüssigkeit gefüllt.

In einer weiteren bevorzugten Ausführungsform des erfindungsgemäßen Verfahrens werden abgeschlossene Reaktionsräume gebildet, insbesondere mit Flüssigkeit gefüllte Reaktionsräume, während die Oberfläche des Sensor-Arrays mit der Oberfläche der Stempelschicht in direktem mechanischen Kontakt steht. Die abgeschlossenen Reaktionsräume werden insbesondere durch Erhöhungen auf der Oberfläche des Sensor-Arrays zwischen den Sensoren, durch die Oberfläche des Sensor-Arrays und durch die Poren der porösen Stempelschicht, welche zur Oberfläche der Stempelschicht hin offen sind, gebildet.

In einer besonders bevorzugten Ausführungsform des erfindungsgemäßen Verfahrens führen die Sensoren elektrochemische Messungen durch, insbesondere Voltammetrische- und/oder Chronoamperometrische- und/oder Coulometrische- und/oder Impedanz-Messungen. Die Messungen der Sensoren können gleichzeitig oder nacheinander erfolgen.

In einer weiteren Ausführungsform des Verfahrens werden die Oberfläche der Stempelschicht, welche dem Sensor-Array gegenüber liegt, und die Oberfläche des Sensor-Arrays parallel zueinander, aufeinander zu bewegt. Dabei werden sie insbesondere vor, nach und zu jedem Zeitpunkt der Bewegung parallel zueinander ausgerichtet. Die Bewegung der Stempelschicht erfolgt bevorzugt in einer Richtung senkrecht zur Oberfläche des Sensor-Arrays. Dabei kann die Stempelschicht in Richtung Sensor-Array bewegt werden, oder das Sensor-Array kann in Richtung Stempelschicht bewegt werden, oder das Sensor-Array und die Stempelschicht können beide gleichzeitig aufeinander zu bewegt werden.

Die Verwendung der zuvor beschriebenen Vorrichtung und/oder des Verfahrens umfasst die Verwendung in einer Chipkarte oder Kassette, insbesondere in einer Einweg-Chipkarte oder Einweg-Kassette, mit einem Chip, z.B. einem CMOS-Chip mit einem Sensor-Array, insbesondere mit elektrochemischen Sensoren, wobei der Chip insbesondere in einer polymeren Vergussmasse, wie z.B. Plastik, eingegossen ist. Die Stempelschicht ist z.B. durch ein Spritzgussverfahren in Aussparungen oder auf die Oberfläche der Einweg-Chipkarte oder Einweg-Kassette auf- bzw. eingebracht.

Dabei kann die Chipkarte in ein Auslesegerät, insbesondere in ein Handheld-Auslesegerät, eingesetzt werden, welches insbesondere eine Spannungs- und/oder Stromquelle, ein Display, eine digitale Schnittstelle für den Chip, eine Signalverarbeitungseinheit, einen elektrischen, besonders bevorzugt einen thermostatisierten elektrischen Abgriff, einen Stempel-Aktuator und/oder Reagenz-Aktuator enthält.

Wird ein Druck vom Auslesegerät auf die Chipkarte ausgeübt, insbesondere über den Stempel flächig, so wird die auf den Chip gedrückte Stempelschicht so in mechanischen Kontakt mit der Chipoberfläche gebracht, das mit zu analysierender Flüssigkeit gefüllte Bereiche, insbesondere Poren, an der Oberfläche der Stempelschicht über unterschiedliche Sensoren hinweg keine Flüssigkeit austauschen. Damit sind abgeschlossene Reaktionsbereiche über den jeweiligen Sensoren ausgebildet.

Die Verwendung der zuvor beschriebenen Vorrichtung und/oder des Verfahrens schließt eine Verwendung in einem immunologischen Schnelltest und/oder in einem Bluttest und/oder in einer DNA oder RNA-Analyse und/oder in einem Antikörpertest und/oder in einem Peptidtest mit ein.

Der Erfindung liegt allgemein die Idee zu Grunde, dass bei Messungen in Flüssigkeiten mit mehreren Sensoren eines Sensor-Arrays über den Sensoren voneinander getrennte Reaktionsräume geschaffen werden. Diese Reaktionsräume entstehen mit Hilfe eines Stempels, welcher an seiner Oberfläche eine poröse Stempelschicht aufweist, die für Flüssigkeiten undurchlässig ist bezogen auf ihre Gesamtdicke. Insbesondere sind auf der Oberfläche des Sensor-Arrays zwischen den Sensoren jeweils Erhöhungen bzw. Wände, welche die Sensoren voneinander, in der Ebene der Oberfläche betrachtet, vollständig trennen bzw. abgrenzen. Der Stempel kann in Richtung Oberfläche des Sensor-Arrays gedrückt werden, wobei sich die Wände in die Stempelschicht eindrücken oder in die Poren der Stempelschicht hineingedrückt werden. Dadurch wird ein möglicher Flüssigkeitsstrom oder Flüssigkeitsaustausch über den Wänden unterbunden. Durch offene Poren an der Oberfläche der Stempelschicht, welche mit Flüssigkeit gefüllt sind, kann direkt über den Sensoren ein Flüssigkeitsaustausch stattfinden. Die Stempelschicht kann auf die Oberfläche des Sensor-Arrays gedrückt werden und auf dieser aufsetzen, ohne dass die Flüssigkeit über den Sensoren vollständig verdrängt wird. Bei Verwendung einer Stempelschicht aus elastischem Material oder von Wänden aus elastischem Material können irreversiblen Deformationen bzw. Beschädigungen der Erhöhungen bzw. Wände beim Aufdrücken der Stempelschicht auf das Sensor-Array verhindert werden.

Damit werden Messungen mit verringerten Messfehlern möglich, ohne störende Flüssigkeitsströmungen über das ganze Sensor-Array hinweg bzw. gleichzeitig über benachbarte Sensoren hinweg. Der technische Aufwand für die Herstellung der Vorrichtung ist verringert bezogen auf Vorrichtungen, bekannt aus dem Stand der Technik mit starren Stempeln und glatten Stempeloberflächen. Es sind größere Fertigungstolleranzen möglich bei der Herstellung der Erhöhungen bzw. Wände, welche die Sensoren voneinander in der Ebene der Oberfläche des Sensor-Arrays voneinander abgrenzen bzw. trennen. Die Vorrichtung und das Messverfahren sind weniger abhängig von der Dimensionierung des Stempeldrucks, mit dem der Stempel auf die Oberfläche des Sensor-Arrays gedrückt wird.

Für das erfindungsgemäße Verfahren und die erfindungsgemäßen Verwendungen ergeben sich die vorstehend erwähnten, mit der erfindungsgemäßen Vorrichtung verbundenen Vorteile.

Bevorzugte Ausführungsformen der Erfindung mit vorteilhaften Weiterbildungen gemäß den Merkmalen der abhängigen Ansprüche werden nachfolgend anhand der folgenden Figuren näher erläutert, ohne jedoch darauf beschränkt zu sein.

Es zeigen:
- Fig. 1: eine schematische Darstellung eines Sensor-Arrays 1 nach dem Stand der Technik vor A) und während B) der Messung, mit zu Sensoren 3 zugeordneten Reaktionsräumen 4 und einem steifen, kompakten Stempel 2 mit glatter, geschlossener Oberfläche, zum Abdichten der Reaktionsräume 4 gegenüber Flüssigkeitsaustausch, und
- Fig. 2: eine schematische Darstellung eines Sensor-Arrays 1 mit Sensoren 3, welche durch Wände 5 voneinander räumlich abgetrennt sind, und einem Stempel 2 mit einer porösen Stempelschicht 9, deren Poren an der Oberfläche mit Flüssigkeit gefüllt sind und deren Poren im Inneren keine Flüssigkeit enthalten, vor C) und während D) des Drückens der Stempelschicht 9 auf die Oberfläche des Sensor-Arrays 1 bzw. vor C) und während D) der Messung, und
- Fig. 3: eine schematische Darstellung der Vorder- E) und Rückseite F) eines Steckmoduls für immunologische Schnelltests,
- Fig. 4: eine schematische Darstellung eines Querschnitts durch das in Fig. 3 gezeigte Steckmodul.

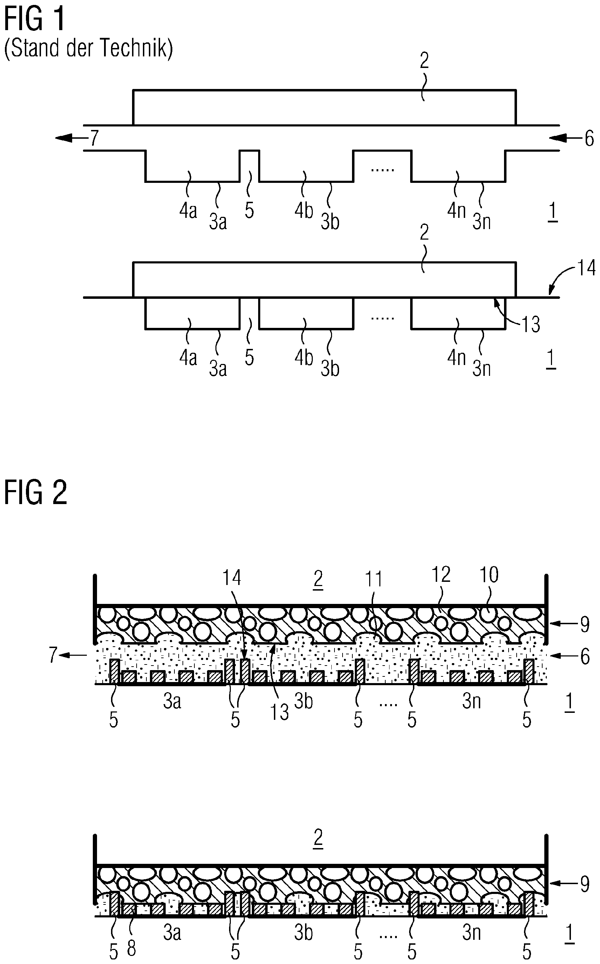

In Fig. 1 ist eine Vorrichtung nach dem Stand der Technik, wie sie z.B. aus der Druckschrift DE 10058394 C1 bekannt ist, im offenen Zustand (Teilfigur A) und im geschlossenen Zustand (Teilfigur B) dargestellt. Die Vorrichtung umfasst ein Sensor-Array 1, welches aus räumlich beabstandeten Sensoren 3a bis 3n aufgebaut ist. Die Sensoren 3a bis 3n sind in Form eines Arrays auf einer planaren Oberfläche angeordnet. Zwischen den Sensoren 3 ragen Wände 5 aus der planaren Oberfläche heraus. Die Wände 5 umgeben die Sensoren 3 vollständig, z.B. in Form von geschlossenen Ringen um jeden einzelnen Sensor 3a bis 3n. Parallel zur planaren Oberfläche ist in einem geringen Abstand, z.B. im Bereich von Mikrometer bis Zentimeter, ein steifer Stempel 2 angeordnet. Mit steifer Stempel 2 ist in diesem Zusammenhang ein Stempel gemeint, welcher aus einem inkompressiblen bzw. nur sehr wenig kompressiblen Material besteht. Im Zwischenraum zwischen dem steifen Stempel 2 und der planaren Oberfläche des Sensor-Arrays 1 mit Wänden 5 kann Flüssigkeit fließen. Dazu können in der planaren Oberfläche benachbart zum Sensor-Array 1 Mikrokanäle ausgebildet sein, welche als Zu- 6 und Abfluss 7 dienen. Wird der Stempel 2 in Richtung planare Oberfläche des Sensor-Arrays 1 gedrückt, so kommt er in direkten mechanischen Kontakt mit den Wänden 5. Sind die Wände 5 so ausgebildet, dass sie gleich hoch aus der planaren Oberfläche ragen und jeden Sensor 3 vollständig umschließen bzw. umfassen, so steht der Stempel 2 bei paralleler Ausrichtung seiner planaren Stempeloberfläche zur planaren Oberfläche des Sensor-Arrays 1 mit allen Wänden 5 gleichzeitig in Kontakt und der Flüssigkeitsstrom ist vollständig unterbunden. Es sind Reaktionsräume 4a bis 4n gebildet, welche mit Flüssigkeit gefüllt sind, und die begrenzt werden durch die planare Oberfläche, in welcher die Sensoren 3a bis 3n angeordnet sind, die Wände 5 und dem Stempel 2. Zwischen verschiedenen Reaktionsräumen 4a bis 4n findet kein Flüssigkeitsaustausch statt. Bei einer Messung mit den Sensoren 3a bis 3n nacheinander oder gleichzeitig, können unterschiedliche Reaktionen in unterschiedlichen Reaktionsräumen 4a bis 4n sich gegenseitig nicht beeinflussen. Ebenfalls kann der unterbundene Flüssigkeitsaustausch zwischen Reaktionsräumen 4a bis 4n zu einer Messung führen, mit geringerem Messfehler im Vergleich zu Messungen bei offenen Reaktionsräumen 4a bis 4n, vergleiche Fig. 1A).

Ein Beispiel für Messungen mit Sensoren 3, bei welchen abgeschlossene Reaktionsräume 4a bis 4n zu einer Verkleinerung von Messfehlern führen, sind elektrochemische Messungen. Die Sensoren 3 im Fall von elektrochemischen Messungen bestehen Beispielsweise aus Metallelektroden 8, z.B. fingerförmig auf der planaren Oberfläche aufgebrachten Goldelektroden. Diese können mit Fängermolekühlen beschichtet sein, spezifisch für die nachzuweisenden Moleküle in der Flüssigkeit. An die Sensoren 3, d.h. Goldelektroden, wird z.B. eine feste Spannung angelegt, und abhängig von an der Oberfläche bindenden Molekülen, ändert sich ein zu messender Stromfluss. Weitere bekannte elektrochemische Methoden sind zyklische Voltammetrie, Chronoamperometrie, Coulometrie, Impedanzspektroskopie, welche sich in den zu steuernden bzw. regelnden Messgrößen und/oder Steuer- bzw. Regelungsmethoden für Strom und Spannung unterscheiden. Den Methoden ist gemeinsam, dass die Messergebnisse stark davon abhängig sind, ob die zu vermessende Flüssigkeit stationär oder als Strömung über den Sensoren 3 vorliegt. Eine einfache Messung, ohne störende Messsignale ist nur bei stationärer oder konstant strömender Flüssigkeit möglich. Der einfachste, technisch zu realisierende Messaufbau besteht in der Realisierung einer stationären Flüssigkeit, wie sie z.B. in der in der Fig. 1B) gezeigten Vorrichtung vorliegt.

Um jedoch eine stationäre Flüssigkeit ohne Strömungen während einer Messung zu realisieren, müssen die Reaktionsräume 4a bis 4n vollständig voneinander getrennt sein. Dies ist nur der Fall, wenn der Stempel 2 gleichzeitig mit allen Wänden 5 in einem engen Kontakt steht, und zwischen keiner Wand und dem Stempel 2 ein Abstand vorliegt. Dies ist nur möglich, wenn alle Wände 5 mit der gleichen Höhe aus der planaren Oberfläche des Sensor-Arrays 1 herausragen. Eine solche Vorrichtung setzt sehr aufwendige Herstellungsverfahren und eine genaue Kontrolle der Maße nach der Herstellung der Vorrichtungen voraus.

In Fig. 2 ist eine Vorrichtung entsprechend einem Ausführungsbeispiel der Erfindung dargestellt. Ein Sensor-Array 1, aufgebaut analog dem zuvor beschriebenen Sensor-Array 1 aus dem Stand der Technik, aus räumlich voneinander getrennten Sensoren 3a bis 3n, ist in einer planaren Oberfläche auf einem Substrat angeordnet. Die Sensoren 3 bestehen z.B. aus fingerförmig angeordneten Goldelektroden. In Fig. 2 ist ein Schnitt durch die Vorrichtung, und somit durch die fingerförmigen Elektroden 8, dargestellt. Weitere Elektrodenmaterialien, welche verwendet werden können, sind Metalle wie z.B. Silber oder Silberchlorid-Paste, Palladium, Kupfer, Platin oder platiniertes Platin, Halbleitermaterialien, wie z.B. Titanoxid oder Silizium, Materialien wie z.B. Graphit, Glascarbon und Verbindungen der genannten Materialien.

Zwischen den Elektroden 8 sind Wände 5 angeordnet, welche die Elektroden 8 räumlich voneinander, zumindest in der planaren Ebene trennen. Die Wände 5 können in Form von flach auf der planaren Oberfläche liegenden geschlossenen Ringen ausgebildet sein, oder in Form von Rechtecksbegrenzungen (analog den Feldbegrenzungen eines Schachbrettes), oder in Form von Dreiecksbegrenzungen, oder in Form einer Wabenstruktur. Strukturen, welche wie ein Netzt die Fläche möglichst vollständig überspannen, sind bevorzugt.

Die Wände 5 sind vorzugsweise aus dem gleichen Material ausgebildet wie das Trägersubstrat, auf welchem die Elektroden 8 angeordnet sind. Mögliche Materialien sind Silizium, Siliziumoxid, Plastik wie z.B. Polyimid, Glas oder Photolack wie z.B. PBO (Polybenzoxazol). Die Erfindung ist aber nicht auf diese Materialien beschränkt. Die Wände 5 können auch aus anderen Materialien als dem Trägermaterial bestehen.

Insbesondere können die Wände 5 aus elastischen oder leicht plastisch verformbaren Materialien bestehen, wie z.B. Kunststoffen, Gummi bzw. Kautschuk oder leicht verformbaren Lacken sowie Dichtungsmaterialien wie z.B. Teflon oder PCTFE (Polychlortrifluorethylen). Bei plastisch schwer verformbaren Materialien der Stempelschicht 9, wie z.B. porösem Siliziumoxid oder porösem Glas, führen die leicht verformbaren Wände zu gut dichtenden Eigenschaften der Vorrichtung bei in Kontaktbringen der Wände 5 mit der porösen Stempelschicht 9.

Die Wände 5 können aus der Oberfläche geätzt oder gestanzt sein, aufgedampft oder gesputtert sein, oder in Form von Photolack aufgebracht sein. Die Erfindung ist aber nicht auf Wände 5 beschränkt, welche nach den zuvor genannten Methoden hergestellt sind. Eine Vielzahl weiterer Methoden ist denkbar. Eine weitere Methode ist das Anordnen der Sensoren 3 in Vertiefungen in der Oberfläche, in einer Fläche parallel zur planaren Oberfläche. Die Wände 5 und das Trägermaterial sind bevorzugt nicht Flüssigkeitsdurchlässig.

Wie in Fig. 2 dargestellt ist, ist gegenüber der planaren Oberfläche des Sensor-Arrays 1 mit Wänden 5 zwischen den Sensoren 3, eine poröse Stempelschicht 9 angeordnet. Im Ausgangzustand ist die poröse Stempelschicht 9 beabstandet zur planaren Oberfläche des Sensor-Arrays 1 und beabstandet zu den Wänden der planaren Oberfläche angeordnet, siehe Figurenteil C). Der Abstand kann im Bereich von Mikrometern, oder im Bereich von Millimetern, oder im Bereich von Zentimetern liegen. Bevorzugt ist die poröse Oberfläche der Stempelschicht 9, ohne Betrachtung der offenen Poren 11, planar und parallel zur planaren Oberfläche des Sensor-Arrays 1 angeordnet.

Der Zwischenraum zwischen der planaren Oberfläche des Sensor-Arrays 1 und der porösen Oberfläche der Stempelschicht 9 kann mit Flüssigkeit befüllt bzw. gefüllt werden. Die Flüssigkeit kann in einem Zufluss 6 zugeführt werden, den Zwischenraum durchströmen und in einem Abfluss 7 abgeführt bzw. entnommen werden.

Beispiele für Flüssigkeiten, die mit der dargestellten Vorrichtung untersucht werden können, sind Körperflüssigkeiten wie Blut oder Urin oder Speichel, deren Bestandteile bzw. Reaktionsprodukte nach z.B. Aufschlussreaktionen nachgewiesen werden. Es können so z.B. DNA-Moleküle bzw. Fragmente mit Hilfe der Sensoren 3 nachgewiesen bzw. analysiert werden, Viren bzw. Antikörper, Peptide oder andere biochemischen Verbindungen wie z.B. Enzym-Substrat-Lösungen können untersucht werden.
Als Flüssigkeiten können aber auch Abwässer mit chemischen Verunreinigungen oder Trinkwasser untersucht werden bzw. andere chemische Substanzen in Flüssigkeiten nachgewiesen werden.

Vor, nach und in Zwischenschritten der Untersuchung der Flüssigkeiten kann mit Spülflüssigkeit, z.B. tridestilliertem Wasser oder Alkohol bzw. anderen Lösungsmitteln, der Zwischenraum und die Oberflächen der Stempelschicht 9 sowie des Sensor-Arrays gereinigt werden. Die Spülflüssigkeit wird einmalig oder mehrmalig durch den Zwischenraum gespült. Eine Reinigung durch spülen mit Luft oder anderen Gasen ist auch möglich. Speziell um Reste der Spülflüssigkeit zu entfernen kann nach einem Spülvorgang mit z.B. Stickstoff der Zwischenraum, die Stempelschicht 9 mit den offenen Poren 11 und die Oberfläche des Sensor-Arrays 1 mit den Sensoren 3 und Wänden 5 getrocknet und gereinigt werden.

Bei Befüllen und Durchströmen des Zwischenraums zwischen Sensor-Array 1 und Stempelschicht 9 mit Flüssigkeit ist vorzugsweise der gesamte Raum des Zwischenraums mit Flüssigkeit ausgefüllt. Luftblasen oder Gasblasen können durch rütteln und spezielle Befüllungsschritte vermieden werden. Auch hydrophile Beschichtungen können zu einer besseren Benetzung und zu einer Vermeidung von Luftblasen beim Befüllen führen. Die Stempelschicht 9 ist in ihrem Inneren nicht mit Flüssigkeit zu befüllen. Das Material der Stempelschicht 12 ist Flüssigkeitsundurchlässig, zumindest für die zu untersuchende Flüssigkeit. Nur die zur Oberfläche hin offenen Poren 11 der Stempelschicht 9 werden mit Flüssigkeit gefüllt. Auf der gegenüberliegenden Seite zur Stempelschicht 9 stehen die Sensoren 3 und die Wände 5 mit der Flüssigkeit in Kontakt. Vorzugsweise ist die gesamte Oberfläche des Sensor-Arrays 1 bzw. der Sensoren 3 mit den auf der Oberfläche befindlichen Wänden 5 vollständig benetzt. An der Oberfläche haftende Luftblasen können Messungen stören oder verhindern.

Die Stempelschicht 9 kann auf einem kompakten Stempel 2 fest oder locker aufgebracht sein oder mit diesem beim drücken der Stempelschicht 9 in Richtung Sensor-Array 1 erst in Kontakt kommen. Ein festes Aufbringen kann in Form von Kleben oder Löten sowie anderen Verbindungsmethoden erfolgen. Der Stempel kann aber auch vollständig aus der Stempelschicht 9 bestehen, oder die Stempelschicht 9 kann z.B. durch ätzen an der Oberfläche des Stempels erzeugt werden. Wesentlich ist ein poröser Aufbau der Stempelschicht mit offenen Poren 11 an der Oberfläche der Stempelschicht 9. Die Stempelschicht 9 kann auch in Form einer Folie gegenüber dem Sensor-Array angebracht sein, mit offenen Poren 11 an der Oberfläche und geschlossenen Poren 10 im Inneren der Stempelschicht 9. Die geschlossenen Poren 10 der Stempelschicht 9 können mit Luft oder einem Gas gefüllt sein.

Vor einer Messung mit den Sensoren 3 des Sensor-Arrays 1 wird die Stempelschicht 9 in Richtung Oberfläche des Sensor-Arrays 1 verschoben. Dies erfolgt bevorzugt durch einen Druck auf die Rückseite der Stempelschicht 9. Die Rückseite ist die Seite der Stempelschicht 9, welche der porösen Oberfläche der Stempelschicht 9, die gegenüber der Oberfläche des Sensor-Arrays 1 angeordnet ist, gegenüber liegt. Die Rückseite steht oder wird in Kontakt gebracht mit dem Stempel 2. Bevorzugt übt der Stempel 2 einen gleichmäßigen Druck flächig auf die gesamte Rückseite der Stempelschicht 9 aus.

Dadurch wird die Stempelschicht 9 in Richtung Oberfläche des Sensor-Arrays 1 bewegt. Bevorzugt erfolgt die Bewegung in einer Form, bei der die poröse Oberfläche 13 der Stempelschicht 9 parallel zur planaren Oberfläche des Sensor-Arrays 1 auf die Oberfläche des Sensor-Arrays 1 zu bewegt wird. Die Bewegung kann gleichmäßig mit fester Geschwindigkeit oder beschleunigt erfolgen.

Durch das Bewegen der Stempelschicht 9 in Richtung Sensor-Array 1 wird ein Teil der Flüssigkeit aus dem Zwischenraum verdängt. Die Flüssigkeit kann über den Abfluss 7 abfließen. Statt der Stempelschicht 9 kann auch das Sensor-Array 1 in Richtung Stempelschicht 9 bewegt werden, oder die Stempelschicht 9 und das Sensor-Arrays 1 können gleichzeitig aufeinander zu bewegt werden.

Die Bewegung erfolgt so lange, bis ein ausreichender Kontakt zwischen der Stempelschicht 9 und dem Sensor-Array 1 mit Sensoren 3 und Wänden 5 besteht. Ist der Druck, der durch den Stempel 2 auf die Rückseite der Stempelschicht 9 ausgeübt wird gleich dem Gegendruck durch das Sensor-Array 1 auf die Stempelschicht 9, dann ist die Bewegung des Sensor-Arrays 1 und der Stempelschicht 9 aufeinander zu beendet.

Nach Beendigung der Bewegung sind Wände 5 in die offenen Poren 11 der Stempelschicht 9 gedrückt und führen zu einer Dichten Verbindung der Wände 5 mit der Stempelschicht 9, über welche kein Flüssigkeitstransfer hinweg mehr stattfinden kann oder zumindest stark eingeschränkt ist. Es entstehen zwischen den Wänden 5 und der Stempelschicht 9 Flüssigkeitsdichte Verbindungen, bevorzugt bei verformbaren Wänden 5 und/oder verformbarer Stempelschicht. Bei ausreichendem Druck können auch Flüssigkeitsdichte Verbindungen entstehen, wenn die Wände 5 und die Stempelschicht 9 aus ähnlich oder gleich starken bzw. unverformbaren Materialien bestehen, oder die Wände 5 aus stärkerem Material bestehen als die Stempelschicht 9. Die Wände 5 werden in diesem Fall in die offenen Poren 11 gedrückt, bis sie das Material der Stempelschicht 9 in den Poren 11 berühren und schneiden bzw. werden gequetscht in die Stege des Materials der Stempelschicht 9, welche einzelne offene Poren 11 voneinander abgrenzen.

Über den Sensoren 3 ist auch nach Beendigung der Bewegung zu untersuchende Flüssigkeit. Die Sensoren 3 stehen mit der Flüssigkeit in Kontakt und können Substanzen in der Flüssigkeit oder Bestandteile der Flüssigkeit Messen und Nachweisen. Chemische Reaktionen, welche in der Flüssigkeit stattfinden, können mit Hilfe der Sensoren 3 zeitlich aufgelöst beobachtet werden. Bei direktem Kontakt der Stempelschicht 9 mit den Sensoren 3 bzw. mit den Elektroden 8 der Sensoren steht die Flüssigkeit in den offenen Poren 11 in Kontakt mit den Sensoren 3. Das Material 12 der Stempelschicht 9 setzt auf den Elektroden 8 auf bzw. steht mit diesen in direktem mechanischem Kontakt.

Auch bei nicht vorhandenen Wänden 5 führt dies zu einer Unterbindung oder starken Verringerung von Flüssigkeitsströmung über den Sensoren 3. Eine Vorrichtung ohne Wände 5 führt somit ebenfalls zu einer Verringerung von Messfehlern bzw. Rauschen bei der Messung.

In einer bevorzugten Ausführungsform sind die Elektroden 8 der Sensoren 3, besonders bevorzugt die Gesamtheit der Elektroden 8 jeweils eines Sensors 3, kleiner als der Durchmesser der Poren 11 der Stempelschicht 9. So können die Elektroden 8 z.B. Fingerförmig sein mit 1µm breiten Fingern, welche in einem Abstand voneinander von 1µm angeordnet sind, und eine Höhe von bis zu 0,5µm aufweisen. Der Sensor 3 besteht z.B. aus zwei kammförmigen, wechselseitig ineinander greifenden Elektroden-Systemen, welche einen etwa 150µm bis 200µm breiten Sensor 3 bilden. Der Sensor 3 ist z.B. zwischen 20µm breiten und 5µm hohen Wänden mittig angeordnet, und ergibt als Gesamtheit einen Kreis mit einem Durchmesser von 150µm, wobei die Wände kreisförmig einen Innenraum mit Sensor 3 von z.B. 200µm Durchmesser umschließen. Eine Pore 11 würde in diesem Ausführungsbeispiel einen Durchmesser zwischen 2µm und 200µm aufweisen, besonders bevorzugt einen Durchmesser, welcher eine Pore 11 über mindestens zwei benachbarte Elektroden sich erstrecken lässt, z.B. im Bereich von 2µm bis 5µm, mit einer Tiefe in die Stempelschicht 9 hinein von in der Größenordnung 5µm bis 200µm. Somit kann eine Pore 11 einen vollständige Reaktionsraum bzw. Reaktionskammer 4 über einem Sensor 3 bilden oder einen Reaktionsraum bzw. Reaktionskammer 4, welcher sich über mindestens zwei benachbarte Elektroden erstreckt. Bei Druck auf die Stempelschicht 9 kann das Volumen der Poren 11 verkleinert werden, und durch die Verringerung des Volumens eine Erhöhung der Konzentration an Reaktionsprodukten erreicht werden. Die Empfindlichkeit der Vorrichtung wird damit erhöht bzw. die Nachweisgrenze verbessert, d.h. die minimale Konzentration der nachzuweisenden Substanzen, welche mit der Vorrichtung nachgewiesen werden können, wird verringert.

Eine vorteilhafte Ausbildung der erfindungsgemäßen Vorrichtung mit Wänden 5, wie sie in Fig. 2 dargestellt ist, führt zur Ausbildung von abgeschlossenen Reaktionsräumen 4. Die abgeschlossenen Reaktionsräume 4 sind nach Beendigung der Bewegung ausgebildet und begrenzt von der Stempelschicht 9 mit offenen Poren 11, dem Sensor-Array 1 mit darauf befindlichen Elektroden 8, und den Wänden 5, welche in der Ebene der Oberfläche des Sensor-Arrays 1 die Sensoren 3 vollständig umgeben bzw. umschließen.
Die abgeschlossenen Reaktionsräume 4 führen, zusätzlich zur Unterbindung von Strömungen der Flüssigkeit über mehrere Sensoren 3 hinweg, zur Entkopplung von Reaktionen in räumlich voneinander getrennten Reaktionsräumen 4. So können in unterschiedlichen Reaktionsräumen 4 Reaktionen unabhängig voneinander ablaufen und mit den in dem jeweiligen Reaktionsraum 4 befindlichen Sensor 3 zeitlich aufgelöst vermessen werden, oder das Reaktionsergebnis vermessen werden, unabhängig vom Reaktionsergebnis in einem benachbarten Reaktionsraum 4.

Nach Beendigung der Bewegung der Stempelschicht 9 bzw. des Sensor-Arrays 1 kann die Stempelschicht 9 in direktem Kontakt mit den Elektroden 8 der Sensoren stehen, oder einen geringen Abstand von diesen aufweisen. So kann die Stempelschicht 9 z.B. mit den Wänden 5 in Kontakt stehen und insbesondere abgeschlossene Reaktionsräume 4 bilden, und zu den Sensoren 3 oder Elektroden 8 der Sensoren 3 einen Abstand aufweisen, der vorzugsweise im Bereich von Mikrometern, oder im Bereich von Millimetern, oder im Bereich von Zentimetern liegt, abgängig von den Dimensionen der Wände 5.

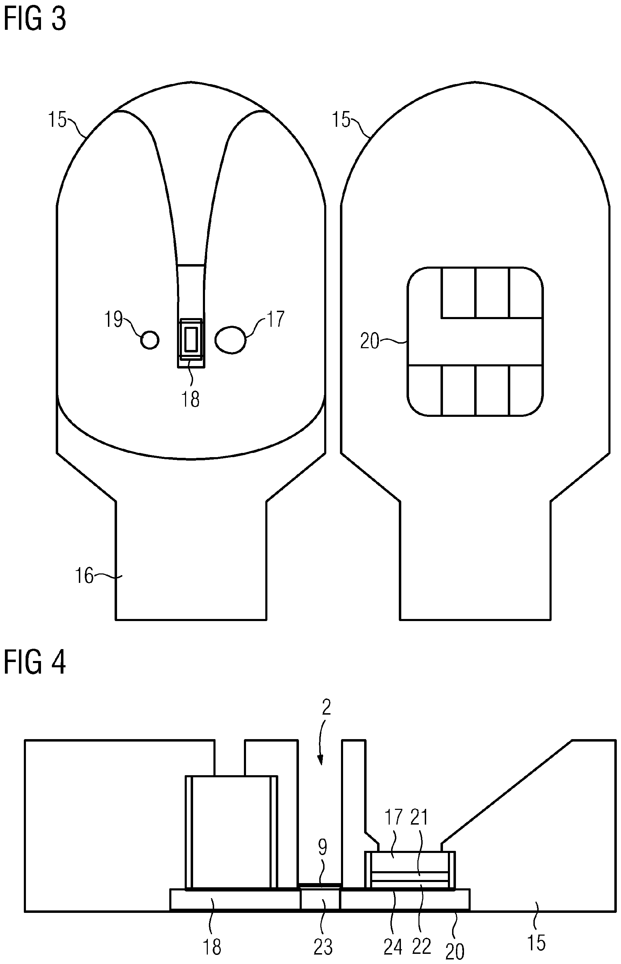

In den Fig. 3 und 4 sind als eine mögliche Anwendung der erfindungsgemäßen Vorrichtung und des erfindungsgemäßen Verfahrens ein Steckmodul für immunologische Schnelltests dargestellt.

Die Fig. 3 zeigt dabei die Vorder- E) und Rückseite F) des Steckmoduls, welches z.B. aus einem Plastik-Körper als Gehäuse 15 besteht, einem darin befindlichen Chip-Modul 18 mit Sensor-Array 1 auf einem Chip 23, elektrischen Kontakten 20, und Mikrokanälen 24 auf dem Chip-Modul 18. Das Steckmodul kann die in Fig. 3 dargestellte Form oder andere Formen, wie z.B. die einer Scheckkarte, aufweisen. In dem Plastikgehäuse 15 sind Kammern und Mikrokanäle für die Aufnahme, den Transport und Reaktionen sowie Abgabe der Flüssigkeit ausgebildet. Ebenfalls Kammern für Reagenzien, wie z.B. Trockenreagenzien, welche für z.B. Aufschluss- und Nachweisreaktionen benötigt werden, können in dem Plastikgehäuse 15 realisiert sein.

Das Gehäuse 15 kann zur besseren Handhabung mit einem Griff 16 und mit Beschriftungen, wie z.B. Patienten- oder Probendaten ausgestattet sein. Alternativ können Daten auf dem Chip 23 gespeichert sein, welcher z.B. ein CMOS-Chip (Complementary Metal Oxide Semiconductor) ist, mit darauf befindlichem Sensor-Array 1 und im Chip 23 integrierter Datenverarbeitungs- und/oder Speichereinrichtung.

Dem Gehäuse 15 wird über einen Zufluss 17 im Gehäuse 15 Flüssigkeit zugeführt. Die Flüssigkeit wird durch die Mikrokanäle 24 über den Chip 23 und das Sensor-Array 1 geleitet. Bevor die Messung beginnt, wird ein Stempel 2 mit Stempelschicht 9 auf den Chip 23 mit Sensor-Array 1 gedrückt. Die Stempelschicht 9 kann sich zuvor beabstandet zum Sensor-Array 1 auf dem Chip 23 befinden, um das Sensor-Array 1 abzudecken und vor Verunreinigungen zu schützen. Oder die Stempelschicht 9 ist fest mit dem Stempel 2 verbunden und wird somit erst mit Einwirkung des Stempels auf das Sensor-Array 1 auf den Chip 23 gedrückt. In diesem Fall ist das Sensor-Array 1 nach oben hin offen, d.h. nicht durch das Gehäuse 15 verkapselt und abgeschlossen gegenüber der Umwelt. Der Stempel 2 mit Stempelschicht 9 wird bis Beendigung der Messung auf den Chip 23 mit Sensor-Array 1 gedrückt. Überschüssige Flüssigkeit sowie Flüssigkeit nach der Messung, wenn der Druck durch den Stempel 2 mit der Stempelschicht 9 aufgehoben ist, kann in einem Abfluss 19 mit Entlüftung entsorgt oder über diesen dem Gehäuse 15 entnommen werden.

Der Stempel 2 ist vorzugsweise in einer nicht dargestellten Messapparatur, welche die Messelektronik umfasst, integriert.

Das Sensor-Array 1 wird über den Chip 23 und elektrische Kontakte 20, welche mit dem Chip 23 elektrisch verbunden sind und sich auf der Gehäuserückseite befinden, kontaktiert. Bei Einführung des Steckmoduls in die Messapparatur wird ein elektrischer Kontakt der Messapparatur mit den elektrischen Kontakten 20 des Steckmoduls hergestellt. Das Sensor-Array 1 kann über die Messelektronik der Messapparatur elektrisch angesprochen werden, und Messgrößen an die Messelektronik übermitteln, welche dann in der Messapparatur verarbeitet und ausgewertet werden. Ein Display oder andere optische sowie akustische Ausgabeeinrichtungen, welche an die Messapparatur angeschlossen sind, können das Messergebnis ausgeben.

In der Fig. 4 ist ein Schnitt durch das in der Fig. 3 gezeigte Steckmodul dargestellt. In dem Gehäuse 15 sind ein Enzym-Substrat-Pad 21, ein Konjugat-Pad 22, das Chip-Modul 18 mit einem Sensor-Array 1 auf z.B. einem CMOS-Chip 23 sowie darauf angeordnete Mikrokanäle 24 integriert. Über einen Flüssigkeits-Zufluss 17 kann die zu untersuchende Flüssigkeit, z.B. Blut, zugeführt werden. Die Flüssigkeit durchfließt beispielsweise das Enzym-Substrat-Pad 21 und das Konjugat-Pad 22. Es können auch weitere Substanz-Pads integriert sein, welche die zu untersuchende Flüssigkeit mit chemischen Substanzen mischen und mit chemischen Reaktionen für die Untersuchung vorbereiten. Beispielsweise kann als Flüssigkeit Blut verwendet werden. Das Blut wird aufgeschlossen, d.h. die Zellen geben Bestandteile wie z.B. DNA frei, welche wiederum in kleine Fragmente zerlegt werden. In einer bevorzugten Ausführungsform werden anschließend die Fragmente an Marker, welche den Nachweis der Fragmente ermöglichen, angebunden.

Die so aufbereitete Flüssigkeit wird über die Mikrokanäle 24 zum Sensor-Array 1 geleitet, d.h. über Kapillarkräfte und/oder einen äußeren Druck bzw. Gravitationskräfte wird die Flüssigkeit von den Mikrokanälen 24 aufgesaugt und/oder werden die Mikrokanäle 24 mit der Flüssigkeit befüllt. Die Flüssigkeit wird über die Mirkokanäle 24 zum Sensor-Array 1 transportiert und über das Sensor-Array 1 geleitet. Eine Öffnung im Gehäuse über dem Chip 23 ermöglicht die Zuführung von Druck in Richtung Chip 23. Der Druck kann in Form von Luftdruck oder direkt mit Hilfe eines Stempels 2 zugeführt werden. Die Stempelschicht 9 kann an dem Stempel 2 befestigt sein oder zuvor auf dem Chip 23 abgelegt worden sein. Die Stempelschicht 9 kann alternativ auch schon beabstandet, über dem Chip 23 angeordnet im Gehäuse 15 integriert sein. In diesem Fall schützt sie den Chip 23 bzw. das Sensor-Array 1 vor Verschmutzung vor, während und nach der Benutzung.

In der in Fig. 4 dargestellten Ausführungsform der erfinderischen Vorrichtung drückt der Stempel 2 über die Öffnung im Gehäuse 15 direkt auf die Stempelschicht 9, welche sich in Richtung Sensor-Array 1 bewegt, bis die Stempelschicht 9 sich in direktem Kontakt mit den Elektroden 8 der Sensoren 3 befindet. Zwischen den Wänden 5 und der Stempelschicht 9 besteht eine Flüssigkeitsdichte Verbindung, womit sich abgeschlossene Reaktionsräume 4 ausgebildet haben, welche mit Flüssigkeit gefüllt sind. Messungen mit verschiedenen Sensoren 3a bis 3n können unabhängig voneinander, gleichzeitig oder nacheinander durchgeführt werden. Flüssigkeitsströmungen über mehrere Reaktionsräume 4 hinweg sind unterbunden und führen zu keinen Störsignalen bei der Messung bzw. zu Messfehlern.
Statt einer direkten Druckausübung durch den Stempel 2 auf die Stempelschicht 9 kann ein Druck auch indirekt über Gehäuseteile 15 auf die Stempelschicht 9 ausgeübt werden, wenn die Stempelschicht 9 vollständig in dem Gehäuse integriert und von diesem Abgedeckt ist.

Nach Ablauf der Reaktionen und den Messungen mit Hilfe der Sensoren 3 wird der Druck auf die Stempelschicht 9 aufgehoben, und die Flüssigkeit kann in einen Flüssigkeits-Abfluss 19 fließen. Sie kann dort verbleiben oder für weitere Untersuchungen entnommen werden. Der Flüssigkeits-Abfluss 19 dient ebenfalls zur Entsorgung von überschüssiger Flüssigkeitsmenge vor den Messungen und für die Entsorgung von z.B. Spülflüssigkeiten, welche vor und nach Messungen zum Spülen des Zwischenraums zwischen Stempelschicht 9 und Sensor-Array 1 verwendet werden.

Das Steckmodul kann für die einmalige Verwendung ausgelegt sein, d.h. als Einwegmodul, oder für die mehrmalige Verwendung.

## Patentansprüche

1. Vorrichtung mit einem Sensor-Array (1) und mit einem Stempel (2) für den Nachweis von Flüssigkeiten oder Substanzen aus Flüssigkeiten in räumlich voneinander getrennten Reaktionsräumen (4), wobei das Sensor-Array (1) aus wenigstens zwei Sensoren (3) besteht, und wobei der Stempel (2) beweglich gegenüber dem Sensor-Array (1) angeordnet ist und eine Stempelschicht (9) an seiner dem Sensor-Array (1) gegenüberliegenden Oberfläche aufweist, welche mit dem Sensor-Array (1) mechanisch kontaktierbar ist,
**dadurch gekennzeichnet,**
**dass** die Stempelschicht (9) zumindest teilweise oder vollständig poröse Eigenschaften aufweist und für Flüssigkeiten oder Substanzen aus Flüssigkeiten impermeabel ist bezogen auf die Gesamtschichtdicke der Stempelschicht (9), wobei die poröse Stempelschicht (9) aus einem Material gebildet ist, welches elastisch verformbar ist, und
**dass** die Stempelschicht (9) offene Poren (11) an der Oberfläche der Stempelschicht (9) aufweist, wobei die Poren (11) einen Durchmesser aufweisen, welcher größer ist als der Abstand von zwei benachbarten Elektroden (8) der Sensoren (3), sodass die Poren (11) jeweils einen Reaktionsraum (4) bilden, welcher sich über mindestens zwei benachbarte Elektroden (8) erstreckt.

2. Vorrichtung nach Anspruch 1, **dadurch gekennzeichnet, dass** die wenigstens zwei Sensoren (3) auf oder in oder unter einer ersten Oberfläche angeordnet sind, insbesondere einer ersten planaren Oberfläche, und eine zweite Oberfläche, insbesondere eine zweite planare Oberfläche, von der Oberfläche der Stempelschicht (9) gebildet ist, welche dem Sensor-Array (1) gegenüber liegt.

3. Vorrichtung nach Anspruch 2, **dadurch gekennzeichnet, dass** der Stempel (2) in einer ersten Position so anordenbar ist, dass die zweite Oberfläche einen Abstand zur ersten Oberfläche aufweist, insbesondere einen Abstand im Bereich von Mikrometern oder Millimetern, und dass der Stempel (2) in einer zweiten Position so anordenbar ist, dass die zweite Oberfläche und die erste Oberfläche in direktem Kontakt stehen.

4. Vorrichtung nach Anspruch 3, **dadurch gekennzeichnet, dass** die Poren (10, 11) der porösen Stempelschicht (9) einen Durchmesser im Bereich von 0,05 bis 200 Mikrometern, insbesondere im Bereich von 1 bis 10 Mikrometern aufweisen, und/oder einen Durchmesser aufweisen, welcher größer ist als der Durchmesser der Sensoren (3).

5. Vorrichtung nach einem der vorhergehenden Ansprüche, **dadurch gekennzeichnet, dass** zwischen den mindestens zwei Sensoren (3) Erhöhungen (5) aus der ersten Oberfläche herausragen, welche insbesondere in der Ebene der ersten Oberfläche die Sensoren (3) jeweils vollständig umgeben oder umschließen, und deren Höhe, mit welcher die Erhöhungen (5) aus der ersten Oberfläche herausragen, größer ist als jegliche Höhe von Strukturen, welche innerhalb von Teilen der ersten Oberfläche angeordnet sind, die von den Erhöhungen (5) umgeben oder umschlossen sind.

6. Vorrichtung nach Anspruch 5, **dadurch gekennzeichnet, dass** die Höhe und/oder Breite der Erhöhungen (5) zwischen den mindestens zwei Sensoren (3) in der Größenordnung der Durchmesser der Poren (11) der Stempelschicht (9) sind, insbesondere im Bereich von 0,05 bis 200 Mikrometern, besonders bevorzugt im Bereich von 1 bis 20 Mikrometern, und/oder dass in der zweiten Position des Stempels (2) die aus der ersten Oberfläche herausragenden Erhöhungen (5) teilweise oder vollständig in den Poren (11) der Stempelschicht (9) anordenbar sind.

7. Vorrichtung nach Anspruch 5 oder 6, **dadurch gekennzeichnet, dass** durch einen direkten Kontakt zwischen den Erhöhungen (5) und der Stempelschicht (9) eine für Gase oder Flüssigkeiten dichte Verbindung besteht und/oder nur ein Gas oder Flüssigkeitsaustausch über die Poren (11) möglich ist, welche direkt über den Sensoren (3) angeordnet sind.

8. Vorrichtung nach einem der vorhergehenden Ansprüche, **dadurch gekennzeichnet, dass** der Raum zwischen dem Sensor-Array (1) und der Stempelschicht (9) teilweise oder vollständig mit Flüssigkeit füllbar oder gefüllt ist und/oder nur Poren (11) an der Oberfläche der porösen Stempelschicht. (9) mit Flüssigkeit füllbar oder gefüllt sind, wobei Poren (10) im inneren der Stempelschicht (9) keine Flüssigkeit enthalten und/oder nicht mit Flüssigkeit füllbar sind.

9. Vorrichtung nach einem der Ansprüche 2 bis 8, **dadurch gekennzeichnet, dass** die wenigstens zwei Sensoren (3) elektrochemische Sensoren (3), insbesondere fingerförmig auf der ersten Oberfläche angeordnete Metallelektroden (8) sind.

10. Vorrichtung nach einem der vorhergehenden Ansprüche, **dadurch gekennzeichnet, dass** die wenigstens zwei Sensoren (3) aus Gold, Silber und/oder Silberchlorid, Platin, Palladium und/oder Kupfer aufgebaut sind oder Verbindungen oder Legierungen dieser Metalle enthalten.

11. Vorrichtung nach einem der Ansprüche 5 bis 10, **dadurch gekennzeichnet, dass** die poröse Stempelschicht (9) aus einem porösen Material gebildet ist, welches porösen Kunststoff, porösen Gummi oder porösen Kautschuk enthält oder daraus gebildet ist.

12. Vorrichtung nach einem der vorhergehenden Ansprüche, **dadurch gekennzeichnet, dass** die Schichtdicke der Stempelschicht (9) im Bereich von Mikrometern ist, oder im Bereich von Millimetern ist, oder im Bereich von Zentimetern ist, und/oder der Stempel (2) vollständig aus der Stempelschicht (9) besteht.

13. Verfahren zum Nachweis von Flüssigkeiten oder Substanzen aus Flüssigkeiten in räumlich voneinander getrennten Reaktionsräumen (4), mit einer Vorrichtung nach einem der vorhergehenden Ansprüche, mit den zeitlich aufeinander folgenden Schritten:
- Befüllen und/oder Durchströmen eines Raumes mit einer Flüssigkeit, welcher gebildet wird vom Zwischenraum zwischen einer Oberfläche des Sensor-Arrays (1) und der Oberfläche der Stempelschicht (9),
- Bewegen oder Drücken der Stempelschicht (9) in Richtung des Sensor-Arrays (1) mit Hilfe eines mit der Stempelschicht (9) in Verbindung stehenden Stempels (2), bis die Oberfläche des Sensor-Arrays (1) mit der Oberfläche der Stempelschicht (9) in direktem mechanischen Kontakt steht, wobei während des direkten Kontaktes zwischen der Oberfläche des Sensor-Arrays (1) und der Oberfläche der Stempelschicht (9) kein Flüssigkeitsaustausch zwischen jedem mit Flüssigkeit gefüllten Bereich direkt über einem ersten Sensor der wenigstens zwei Sensoren (3) des Sensor-Arrays (1) und jedem mit Flüssigkeit, gefüllten Bereich direkt über dem wenigstens einem zweiten Sensor stattfndet,
- Messen mit den Sensoren (3) des Sensor-Arrays (1).

14. Verfahren nach Anspruch 13, **dadurch gekennzeichnet, dass** die mit Flüssigkeit befüllten Bereiche über den Sensoren (3) ausschließlich durch Bereiche gebildet werden, welche aus mit Flüssigkeit gefüllten Poren (11) an der Oberfläche der porösen Stempelschicht (9) bestehen und Poren (10) nicht mit Flüssigkeit gefüllt werden, welche nicht in Verbindung stehen mit der Oberfläche der Stempelschicht (9) und im inneren der porösen Stempelschicht (9) angeordnet sind.

15. Verfahren nach Anspruch 14, **dadurch gekennzeichnet, dass** abgeschlossene Reaktionsräume (4), insbesondere mit Flüssigkeit gefüllte Reaktionsräume (4), gebildet werden während die Oberfläche des Sensor-Arrays (1) mit der Oberfläche der Stempelschicht (9) in direktem mechanischen Kontakt steht, durch Erhöhungen (5) auf der Oberfläche des Sensor-Arrays (1) zwischen den Sensoren (3), die Oberfläche des Sensor-Arrays (1) und Poren (11) der porösen Stempelschicht (9), welche zur Oberfläche der Stempelschicht (9) hin offen sind.

16. Verfahren nach einem der Ansprüche 13 bis 15, **dadurch gekennzeichnet, dass** die Sensoren (3) elektrochemische Messungen durchführen, insbesondere Voltammetrische- und/oder Chronoamperometrische- und/oder Coulometrische-und/oder Impedanz-Messungen.

17. Verfahren nach einem der Ansprüche 13 bis 16, **dadurch gekennzeichnet, dass** die Messungen der Sensoren (3) gleichzeitig oder nacheinander erfolgen.

18. Verfahren nach einem der Ansprüche 13 bis 17, **dadurch gekennzeichnet, dass** die Oberfläche der Stempelschicht (9), welche dem Sensor-Array (1) gegenüber liegt, und die Oberfläche des Sensor-Arrays (1) parallel zueinander aufeinander zu bewegt werden, insbesondere vor, nach und zu jedem Zeitpunkt der Bewegung parallel zueinander ausgerichtet werden.

19. Verfahren nach einem der Ansprüche 13 bis 18, **dadurch gekennzeichnet, dass** die Bewegung der Stempelschicht (9) in einer Richtung senkrecht zur Oberfläche des Sensor-Arrays (1) erfolgt.

20. Verfahren nach einem der Ansprüche 13 bis 19, **dadurch gekennzeichnet, dass** die Stempelschicht (9) in Richtung Sensor-Array (1) bewegt wird, oder dass das Sensor-Array (1) in Richtung Stempelschicht (9) bewegt wird, oder dass das Sensor-Array (1) und die Stempelschicht (9) beide gleichzeitig aufeinander zu bewegt werden und/oder dass bei mechanischem Kontakt zwischen der Stempelschicht (9) und dem Sensor-Array (1) die Stempelschicht (9) so komprimiert wird, dass sich das Volumen von Poren (11) an der Oberfläche der Stempelschicht (9) verkleinert.

21. Verwendung der Vorrichtung nach einem der Ansprüche 1 bis 12 und/oder des Verfahrens nach einem der Ansprüche 13 bis 20 in einer Chipkarte oder Kassette, insbesondere in einer Einweg-Chipkarte oder Einweg-Kassette, mit einem Chip (23), insbesondere einem CMOS-Chip mit einem Sensor-Array (1), insbesondere mit elektrochemischen Sensoren (3), wobei der Chip (23) insbesondere in einer polymeren Vergussmasse, insbesondere Plastik, eingegossen ist und/oder die Stempelschicht (9) durch insbesondere Spritzgussverfahren in Aussparungen oder auf die Oberfläche der Chipkarte oder Kassette auf- oder eingebracht ist.

22. Verwendung nach Anspruch 21, **dadurch gekennzeichnet, dass** die Chipkarte oder Kassette in ein Auslesegerät, insbesondere in ein Handheld-Auslesegerät, eingesetzt wird, welches insbesondere eine Spannungs- und/oder Stromquelle, ein Display, eine digitale Schnittstelle für den Chip (23), eine Signalvererbeitungseinheit, einen elektrischen, besonders bevorzugt einen thermostatisierten elektrischen Abgriff, einen Stempel-Aktuator und/oder Reagenz-Aktuator enthält.

23. Verwendung nach Anspruch 22, **dadurch gekennzeichnet, dass** ein Druck vom Auslesegerät auf die Chipkarte oder Kassette, insbesondere über den Stempel (2) flächig, ausgeübt wird und die auf den Chip (23) gedrückte Stempelschicht (9) so in mechanischen Kontakt mit der Chipoberfläche gebracht wird, das mit zu analysierender Flüssigkeit gefüllte Bereiche, insbesondere Poren (11), an der Oberfläche der Stempelschicht (9) über unterschiedliche Sensoren (3) hinweg keine Flüssigkeit austauschen, womit abgeschlossene Reaktionsbereiche über den jeweiligen Sensoren (3) ausgebildet werden und/oder Porenvolumina verkleinert werden.

24. Verwendung nach einem der Ansprüche 20 bis 23 in einem immunologischen Schnelltest und/oder in einem Bluttest und/oder in einer DNA oder RNA-Analyse und/oder in einem Antikörpertest und/oder in einem Peptidtest und/oder Enzym-Substrat-Test.

## Claims

1. Apparatus with a sensor array (1) and with a plunger (2) for the detection of liquids or substances from liquids in spatially separated reaction spaces (4), wherein the sensor array (1) consists of at least two sensors (3), and wherein the plunger (2) is arranged to be movable relative to the sensor array (1) and comprises a plunger layer (9) on its surface laying opposite the sensor array (1) which is capable of being brought into mechanical contact with the sensor array (1),
**characterised in**
**that** the plunger layer (9) comprises at least partially or completely porous properties and is impermeable for liquids or substances from liquids relative to the total layer thickness of the plunger layer (9), wherein the porous plunger layer (9) is formed from a material which is elastically deformable, and
**that** the plunger layer (9) comprises open pores (11) at the surface of the plunger layer (9), wherein the pores (11) comprise a diameter which is greater than the distance of two adjacent electrodes (8) of the sensors (3), such that the pores (11) each form a reaction space (4) which extends over at least two adjacent electrodes (8).

2. Apparatus according to claim 1, **characterised in that** the at least two sensors (3) are arranged on or in or under a first surface, in particular a first planar surface, and a second surface, in particular a second planar surface, by which the surface of the plunger layer (9) is formed lies opposite the sensor array (1).

3. Apparatus according to claim 2, **characterised in that** the plunger (2) is arrangeable a first position such that the second surface comprises a distance to the first surface, in particular a distance in the range of micrometers or millimeters, and that the plunger (2) is arrangeable in a second position such, that the second surface and the first surface are in direct contact.

4. Apparatus according to claim 3, **characterised in that** the pores (10, 11) of the porous plunger layer (9) comprise a diameter in the range of 0.05 to 200 micrometers, in particular in the range of 1 to 10 micrometers, and/or comprise a diameter which is greater than the diameter of the sensors (3).

5. Apparatus according to one of the preceding claims, **characterised in that** elevations (5) protrude from the first surface between the at least two sensors (3), the elevations (5) surrounding or enclosing each of the sensors (3) completely, in particular in the plane of the first surface, and the height by which the elevations (5) protrude from the first surface being larger than any height of structures which are arranged within parts of the first surface that are surrounded or enclosed by the elevations (5).

6. Apparatus according to claim 5, **characterised in that** the height and/or the width of the elevations (5) between the at least two sensors (3) is in the order of magnitude of the diameters of the pores (11) of the plunger layer (9), in particular in the range of 0.05 to 200 micrometers, particularly preferred in the range of 1 to 20 mirometers, and/or that in the second position of the plunger (2) the elevations (5) protruding from the first surface are partially or completely arrangeable in the pores (11) of the plunger layer (9).

7. Apparatus according to claim 5 or 6, **characterised in that** by a direct contact between the elevations (5) and the plunger layer (9) a gas-tight or liquid-tight connection exits and/or an exchange of gas or liquid is only possible via the pores (11) which are arranged directly above the sensors (3).

8. Apparatus according to one of the preceding claims, **characterised in that** the space between the sensor array (1) and the plunger layer (9) is partially or completely filled or fillable with liquid and/or only pores (11) at the surface of the porous plunger layer (9) are fillable or filled with liquid, wherein pores (10) inside the plunger layer (9) do not contain liquid and/or are not fillable with liquid.

9. Apparatus according to one of claims 2 to 8, **characterised in that** the at least two sensors (3) are electrochemical sensors (3), in particular metal electrodes (8) arranged in finger-shaped fashion on the first surface.

10. Apparatus according to one of the preceding claims, **characterised in that** the at least two sensors (3) are constructed from gold, silver and/or silver chloride, platinum, palladium and/or copper or contain compounds or alloys of said metals.

11. Apparatus according to one of claims 5 to 10, **characterised in that** the porous plunger layer (9) is formed from a porous material which contains or is formed from porous plastic, porous rubber or porous natural rubber.

12. Apparatus according to one of the preceding claims, **characterised in that** the layer thickness of the plunger layer (9) is in the range of micrometers or in the range of millimeters or in the range of centimeters and/or the plunger (2) is completely composed of the plunger layer (9).

13. Method for detecting liquids or substances from liquids in spatially separated reaction spaces (4), with an apparatus according to one of the preceding claims, with the following temporally successive steps:
- filing a space with liquid and/or flowing of a liquid trough a space, the space being formed by the interspace between a surface of the sensor array (1) and the surface of the plunger layer (9),
- moving or pressing the plunger layer (9) in the direction of the sensor array (1) with aid of a plunger (2) which is in connection with the plunger layer (9) until the surface of the sensor array (1) is in direct mechanical contact with the surface of the plunger layer (9), wherein during the direct contact between the surface of the sensor array (1) and the surface of the plunger layer (9) no exchange of liquid takes place between each liquid-filled region directly above a first sensor of the at least two sensors (3) of the sensor array (1) and each liquid-filled region directly above the at least one second sensor,
- measuring with the sensors (3) of the sensor array (1).

14. Method according to claim 13, **characterised in that** the liquid-filled regions above the sensors (3) are formed exclusively by regions which are composed of liquid-filled pores (11) at the surface of the porous plunger layer (9) and pores (10) which are not in connection with the surface of the plunger layer (9) and are arranged inside the porous plunger layer (9) are not filled with liquid.

15. Method according to claim 14, **characterised in that** closed-off reaction spaces (4), in particular liquid-filled reaction spaces (4), are formed while the surface of the sensor array (1) is in direct mechanical contact with the surface of the plunger layer (9), by elevations (5) on the surface of the sensor array (1) between the sensors (3), the surface of the sensor array (1) and pores (11) of the porous plunger layer (9) which are open toward the surface of the plunger layer (9).

16. Method according to one of claims 13 to 15, **characterised in that** the sensors (3) carry out electrochemical measurements, in particular voltametric and/or chronoamperometric and/or coulometric and/or impedance measurements.

17. Method according to one of claims 13 to 16, **characterised in that** the measurements of the sensors (3) are effected simultaneously or successively.

18. Method according to one of claims 13 to 17, **characterised in that** the surface of the plunger layer (9) which lies opposite the sensor array (1) and the surface of the sensor array (1) are moved toward one another parallel to one another are oriented parallel to one another, in particular before, after and at every instant of the movement.

19. Method according to one of claims 13 to 18, **characterised in that** the movement of the plunger layer (9) is in a direction perpendicular to the surface of the sensor array (1).

20. Method according to one of claims 13 to 19, **characterised in that** the plunger layer (9) is moved in direction of the sensor array (1), or that the sensor array (1) is moved in direction of the plunger layer (9), or that the sensor array (1) and the plunger layer (9) are both moved toward one another simultaneously and/or that in case of mechanical contact between the plunger layer (9) and the sensor array (1) the plunger layer (9) is compressed in such a fashion that the volume of the pores (11) at the surface of the plunger layer (9) reduces.

21. Use of the apparatus according to one of claims 1 to 12 and/or the method according to one of claims 13 to 20 in a smart card or cassette, in particular in a disposable smart card or disposable cassette, with a chip (23), in particular a CMOS chip with a sensor array (1), in particular with electrochemical sensors (3), wherein the chip (23) is cast in particular in a polymeric potting compound, in particular plastic, and/or the plunger layer (9) is applied or introduced in particular by means of an injection-molding method into cutouts or onto the surface of the smart card or cassette.

22. Use according to claim 21, **characterised in that** the smart card or cassette is inserted into a read-out device, in particular a handheld read-out device, which contains in particular a voltage and/or current source, a display, a digital interface for the chip (23), a signal processing unit, an electrical, particularly preferred a thermostatically regulated electrical tap, a plunger actuator and/or reagent actuator.

23. Use according to claim 22, **characterised in that** a pressure is exerted by the read-out device on the smart card or cassette, in particular areally via the plunger (2), and the plunger layer (9) pressed on the chip (23) is brought into mechanical contact with the chip surface in a such a way that regions filled with liquid to be analyzed, in particular pores (11), at the surface of the plunger layer do not exchange liquid across different sensors (3), whereby closed-off reaction spaces are formed above the respective sensors (3) and/or pore volumes are reduced.

24. Use according to one of claims 20 to 23 in a immunological fast test and/or in a blood test and/or in a DNA or RNA analysis and/or in an antibody test and/or in a peptide test and/or enzyme substrate test.

## Revendications

1. Dispositif comprenant un réseau de capteurs (1) et comprenant un piston (2) pour attester de la présence de liquides ou de substances composées de liquides dans des chambres de réaction (4) séparées les unes des autres, dans lequel le réseau de capteurs (1) est constitué d'au moins deux capteurs (3), et dans lequel le piston (2) est disposé de manière mobile par rapport au réseau de capteurs (1) et présente une couche de piston (9) au niveau de sa surface faisant face au réseau de capteurs (1), laquelle peut être mise en contact de manière mécanique avec le réseau de capteurs (1),
**caractérisé**
**en ce que** la couche de piston (9) présente au moins en partie ou en totalité des propriétés poreuses et est imperméable aux liquides ou aux substances composées de liquides par rapport à ['épaisseur de couche totale de la couche de piston (9), dans lequel la couche de piston (9) poreuse est formée à partir d'un matériau, qui peut être déformé élastiquement, et
**en ce que** la couche de piston (9) présente des pores (11) ouverts au niveau de la surface de la couche de piston (9), dans lequel les pores (11) présentent un diamètre, qui est plus grand que la distance entre deux électrodes (8) adjacentes aux capteurs (3) si bien que les pores (11) forment respectivement une chambre de réaction (4), qui s'étend au-dessus au moins des deux électrodes (8) adjacentes.

2. Dispositif selon la revendication 1, **caractérisé en ce que** les au moins deux capteurs (3) sont disposés sur ou dans ou sous une première surface, en particulier une première surface plane, et **en ce qu'**une deuxième surface, en particulier une deuxième surface plane, est formée par la surface de la couche de piston (9), qui fait face au réseau de capteurs (1).

3. Dispositif selon la revendication 2, **caractérisé en ce que** le piston (2) peut être disposé dans une première position de telle manière que la deuxième surface présente par rapport à la première surface une distance, en particulier une distance de l'ordre du micromètre ou du millimètre, et **en ce que** le piston (2) peut être disposé dans une deuxième position de telle manière que la deuxième surface et la première surface sont en contact direct.

4. Dispositif selon la revendication 3, **caractérisé en ce que** les pores (10, 11) de la couche de piston (9) poreuse présentent un diamètre situé dans la plage allant de 0,05 à 200 micromètres, en particulier situé dans la plage allant de 1 à 10 micromètres, et/ou présentent un diamètre qui est plus grand que le diamètre des capteurs (3).

5. Dispositif selon l'une quelconque des revendications précédentes, **caractérisé en ce que**, entre les au moins deux capteurs (3), des parties surélevées (5) dépassent de la première surface, ces parties entourant ou renfermant respectivement en totalité les capteurs (3) en particulier dans le plan de la première surface, et **caractérisé en ce que** la hauteur de laquelle les parties surélevées (5) dépassent de la première surface, est plus grande que la hauteur précisément de structures, qui sont disposées à l'intérieur de parties de la première surface, qui sont entourées ou renfermées par les parties surélevées (5).

6. Dispositif selon la revendication 5, **caractérisé en ce que** la hauteur et/ou la largeur des parties surélevées (5) entre les au moins deux capteurs (3) sont de l'ordre de grandeur des diamètres des pores (11) de la couche de piston (9), en particulière sont situées dans la plage allant de 0,05 à 200 micromètres, de manière particulièrement préférée dans la plage allant de 1 à 20 micromètres, et/ou **en ce que** les parties surélevées (5) dépassant de la première surface peuvent être disposées, dans la deuxième position du piston (2), en partie ou en totalité dans les pores (11) de la couche de piston (9).

7. Dispositif selon la revendication 5 ou 6, **caractérisé en ce qu'**une liaison étanche à des gaz ou à des liquides existe du fait d'un contact direct entre les parties surélevées (5) et la couche de piston (9), et/ou **en ce que** seul un échange de gaz ou de liquide est possible par l'intermédiaire des pores (11), qui sont disposés directement au-dessus des capteurs (3).

8. Dispositif selon l'une quelconque des revendications précédentes, **caractérisé en ce que** l'espace entre le réseau de capteurs (1) et la couche de piston (9) peut être remplie ou est remplie en partie ou en totalité de liquide, et/ou **en ce que** seuls des pores (11) au niveau de la surface de la couche de piston (9) poreuse peuvent être remplis ou sont remplis de liquide, dans lequel des pores (10) à l'intérieur de la couche de piston (9) ne contiennent aucun liquide et/ou ne peuvent être remplis de liquide.

9. Dispositif selon l'une quelconque des revendications 2 à 8, **caractérisé en ce que** les au moins deux capteurs (3) sont des capteurs (3) électrochimiques, en particulier sont des électrodes métalliques (8) disposées de manière à présenter une forme de doigt sur la première surface.

10. Dispositif selon l'une quelconque des revendications précédentes, **caractérisé en ce que** les au moins deux capteurs (3) sont constitués d'or, d'argent et/ou de chlorure d'argent, de platine, de palladium et/ou de cuivre ou contiennent des composés ou des alliages desdits métaux.

11. Dispositif selon l'une quelconque des revendications 5 à 10, **caractérisé en ce que** la couche de piston (9) poreuse est formée à partir d'un matériau poreux, qui contient une matière plastique poreuse, une gomme poreuse ou un caoutchouc poreux ou qui en est formé.

12. Dispositif selon l'une quelconque des revendications précédentes, **caractérisé en ce que** l'épaisseur de couche de la couche de piston (9) est de l'ordre du micromètre ou de l'ordre du millimètre ou de l'ordre du centimètre, et/ou **en ce que** le piston (2) est constitué en totalité de la couche de piston (9).

13. Procédé servant à attester la présence de liquides ou de substances composées de liquides dans des chambres de réaction (4) séparées les unes des autres, à l'aide d'un dispositif selon l'une quelconque des revendications précédentes, comprenant les étapes suivantes se suivant de manière chronologique:
- le remplissage et/ou la traversée d'un espace par un liquide, qui est formé par la chambre intermédiaire entre une surface du réseau de capteurs (1) et la surface de la couche de piston (9) ;
- le déplacement ou l'enfoncement de la couche de piston (9) en direction du réseau de capteurs (1) à l'aide d'un piston (2) en liaison avec la couche de piston (9) jusqu'à ce que la surface du réseau de capteurs (1) soit en contact direct mécanique avec la surface de la couche de piston (9), dans lequel aucun échange de liquide entre chaque zone remplie de liquide directement par l'intermédiaire d'un premier capteur des au moins deux capteurs (3) du réseau de capteurs (1) et chaque zone remplie du liquide directement par l'intermédiaire de l'au moins un deuxième capteur n'a lieu au cours du contact direct entre la surface du réseau de capteurs (1) et la surface de la couche de piston (9) ;
- la mesure à l'aide des capteurs (3) du réseau de capteurs (1).

14. Procédé selon la revendication 13, **caractérisé en ce que** les zones remplies de liquide sont formées au-dessus des capteurs (3) exclusivement par des zones, qui sont constituées de pores (11) remplis de liquide au niveau de la surface de la couche de piston (9) poreuse, et **en ce que** des pores (10), qui ne sont pas en liaison avec la surface de la couche de piston (9) et qui sont disposés à l'intérieur de la couche de piston (9) poreuse, ne sont pas remplis de liquide.

15. Procédé selon la revendication 14, **caractérisé en ce que** des chambres de réaction (4) fermées, en particulier des chambres de réaction (4) remplies de liquide, sont formées, alors que la surface du réseau de capteurs (1) est en contact direct mécanique avec la surface de la couche de piston (9), par des parties surélevées (5) sur la surface du réseau de capteurs (1) entre les capteurs (3), par la surface du réseau de capteurs (1) et par les pores (11) de la couche de piston (9) poreuse, qui sont ouverts en direction de la surface de la couche de piston (9).

16. Procédé selon l'une quelconque des revendications 13 à 15, **caractérisé en ce que** les capteurs (3) exécutent des mesures électrochimiques, en particulier des mesures voltamétriques et/ou chronoampérométriques et/ou coulométriques et/ou d'impédance.

17. Procédé selon l'une quelconque des revendications 13 à 16, **caractérisé en ce que** les mesures des capteurs (3) sont effectuées de manière simultanée ou les unes après les autres.

18. Procédé selon l'une quelconque des revendications 13 à 17, **caractérisé en ce que** la surface de la couche de piston (9), qui fait face au réseau de capteurs (1), et la surface du réseau de capteurs (1) sont déplacées l'une vers l'autre de manière parallèle l'une par rapport à l'autre, en particulier sont orientées de manière parallèle l'une par rapport à l'autre avant, après et à chaque moment du déplacement.

19. Procédé selon l'une quelconque des revendications 13 à 18, **caractérisé en ce que** le déplacement de la couche de piston (9) est effectué dans une direction de manière perpendiculaire par rapport à la surface du réseau de capteurs (1).

20. Procédé selon l'une quelconque des revendications 13 à 19, **caractérisé en ce que** la couche de piston (9) est déplacée en direction du réseau de capteurs (1), ou en ce que le réseau de capteurs (1) est déplacé en direction de la couche de piston (9), ou **en ce que** le réseau de capteurs (1) et la couche de piston (9) sont déplacés l'un vers l'autre tous les deux de manière simultanée, et/ou **en ce qu'**en cas de contact mécanique entre la couche de piston (9) et le réseau de capteurs (1), la couche de piston (9) est comprimée de telle manière que le volume de pores (11) se réduit au niveau de la surface de la couche de piston (9).

21. Utilisation du dispositif selon l'une quelconque des revendications 1 à 12 et/ou du procédé selon l'une quelconque des revendications 13 à 20 dans une carte à puce ou une cassette, en particulier dans une carte à puce à usage unique ou dans une cassette à usage unique, avec une puce (23), en particulier une puce CMOS comprenant un réseau de capteurs (1), en particulier comprenant des capteurs (3) électrochimiques, dans laquelle la puce (23) est coulée en particulier dans une masse de scellement polymère, en particulier dans du plastique, et/ou en ce que la couche de piston (9) est appliquée ou introduite, par en particulier un procédé de moulage par injection, dans des évidements ou sur la surface de la carte à puce ou de la cassette.

22. Utilisation selon la revendication 21, **caractérisée en ce que** la carte à puce ou la cassette est insérée dans un appareil de lecture, en particulier dans un appareil de lecture portatif, qui contient en particulier une source de tension et/ou de courant, un écran, une interface numérique pour la puce (23), une unité de traitement de signaux, une prise électrique, de manière particulièrement préférée une prise électrique thermostatée, un actionneur de piston et/ou un actionneur réactif.

23. Utilisation selon la revendication 22, **caractérisée en ce qu'**une pression est exercée par l'appareil de lecture sur la carte à puce ou la cassette, en particulier par l'intermédiaire du piston (2) à plat, et **en ce que** la couche de piston (9) enfoncée sur la puce (23) est amenée en contact mécanique avec la surface de puce de telle manière que des zones remplies de liquide à analyser, en particulier des pores (11), au niveau de la surface de la couche de piston (9) n'échangent aucun liquide au-delà des différents capteurs (3), ce qui permet de réaliser des zones de réaction fermées au-dessus des capteurs (3) respectifs et/ou ce qui permet de réduire des volumes de pores.

24. Utilisation selon l'une quelconque des revendications 20 à 23 lors d'un test rapide immunologique et/ou lors d'un test sanguin et/ou lors d'une analyse d'ADN ou de RNA et/ou lors d'un test des anticorps et/ou lors d'un test de peptide et/ou lors d'un test de substrat enzymatique.
